# EUROPEAN PATENT APPLICATION

(11) **EP 4 700 785 A1**
(43) Date of publication of application: **25.02.2026**
(21) Application number: 24195517.8
(22) Date of filing: 21.08.2024
(51) Int. Cl.: G16H 40/63, A61B 90/70

(54) **MEDICAL OR DENTAL SANITIZING AND/ OR MAINTENANCE DEVICE HAVING AN ARTIFICIAL INTELLIGENCE MODULE**

(71) Applicant: W & H Sterilization S.r.l., 24060 Brusaporto (BG) (IT)
(72) Inventor: BORGHI, Alberto, 25030 Castel Mella (BS) (IT); FACAGNI, Simone, 24060 Brusaporto (BG) (IT); GERVASONI, Mattia, 24010 Sorisole (BG) (IT); MAGNO, Luigi, Marino, 24031 Almenno San Salvatore (IT); MÜNZER, Bernd, 9020 Klagenfurt (AT); PRIMUS, Jürgen, 9586 Fürnitz (AT); MAIER, Klaus, 5020 Salzburg (AT)
(74) Representative: Benda, Ralf

(57) **Abstract**

A medical or dental sanitizing and/ or maintenance device (1) for sanitizing and/ or maintaining a medical or dental appliance (7), having: an imaging device (5) to capture images of the appliance (7), and a trained artificial intelligence module (6) to recognize the appliance (7) based on the images from the imaging device (5) and to output data on the recognized appliance (7), so that the control unit (4) controls a sanitizing and/ or maintaining procedure of the sanitizing and/ or maintenance device (1) based on the provided output data. Alternatively, or in addition, the trained artificial intelligence module (6) determines the soiling of the appliance (7) based on the images and outputs data on the soiling, so that the control unit (4) controls a sanitizing and/ or maintaining procedure of the sanitizing and/ or maintenance device (1) based on the data on the soiling.

## Description

The present invention relates to a medical or dental sanitizing and/ or maintenance device for sanitizing and/ or maintaining a medical or dental appliance having an artificial intelligence module which is trained to provide with data on the medical or dental appliance to be sanitized and/ or maintained and/ or with data on the soling of a medical or dental appliance to be sanitized and/ or maintained, so that the control unit controls the medical or dental sanitizing and/ or maintenance device, in particular a medical or dental sanitizing and/ or maintenance procedure, based on these data.

Patent application EP 2 246 010 A1 discloses a medical or dental sanitizing and/ or maintenance device. The medical or dental sanitizing and/ or maintenance device comprises a coil or an antenna of an RFID read and/or write device to contactlessly read data, e.g., data that identify medical or dental appliances, from data storage units of radio frequency identification (RFID) tags on the medical or dental appliances to be sanitized or maintained.

A disadvantage of the data acquisition disclosed in EP'010 is the variability of the reading range of the RFID reader, since the reading range of an RFID reader can be affected by metallic components and is therefore not constant. E.g., if an RFID reader is positioned close to a swiveling door of a medical or dental sanitizing and/ or maintenance device that comprises metallic elements, depending on the swiveling position of the door the shape of the reading range changes. Accordingly, due to this variability of the reading range on the one hand a user can never be sure whether a medical or dental appliance that he/she wants to be registered by the RFID reader is actually detected. And on the other hand, depending on the current extension of the reading range, an RFID-tag of a medical or dental appliance that is close to the sanitizing and/ or maintenance device but is not put into it may be read by the RFID reader.

Another disadvantage of an RFID based data communication system is that the RFID-tag cannot provide current data, only old data that were previously stored on the label.

It is thus an object of the present application to provide an alternative, more reliable data acquisition for a medical or dental appliance. In particular, the risk of capturing data from a medical or dental appliance not to be treated by the medical or dental sanitizing and/ or maintenance device should be reduced. Alternatively, or in addition, it should be possible to provide current data on medical or dental appliances.

These objects are achieved by a medical or dental sanitizing and/ or maintenance device for sanitizing and/ or maintaining a medical or dental appliance according to Claims 1 and 16, a computer implemented method according to Claims 13 and 28, and a computer program product or computer-readable storage medium according to Claims 15 and 30. Particularly advantageous embodiments are specified in the dependent claims.

The medical or dental sanitizing and/ or maintenance device for sanitizing and/ or maintaining a medical or dental appliance comprises: an enclosure to accommodate the medical or dental appliance to be sanitized and/ or maintained, a feeding device for feeding a sanitizing and/ or maintaining agent into the enclosure to sanitize or maintain the medical or dental appliance, a control unit to control operation of the medical or dental sanitizing and/ or maintenance device, an imaging device which is configured and arranged to capture one or more images of the medical or dental appliance to be sanitized and/ or maintained, and an artificial intelligence module.

According to the instant document the term "medical or dental sanitizing and/ or maintenance device for sanitizing and/ or maintaining a medical or dental appliance" refers to any medical or dental device which is configured to sanitize, sterilize, disinfect, clean, reprocess, rinse and/ or blow with a fluid (gas or liquid), maintain and/ or lubricate a medical or dental appliance. In the following embodiments of such medical or dental sanitizing and/ or maintenance devices for sanitizing and/ or maintaining a medical or dental appliance are listed, without any claim to be exhaustive. The medical or dental sanitizing and/ or maintenance device for sanitizing and/ or maintaining a medical or dental appliance may comprise:
an autoclave or sterilizer; the autoclave or sterilizer preferably applies at least one of steam, radiation, in particular UV-radiation, or a chemical agent, e.g., ethylene oxide or hydrogen peroxide, to sanitize or sterilize a medical or dental appliance;
a thermal cleaning and disinfecting device or thermal washer disinfector (TWD); the TWD applies at least one of water, steam, or a detergent to sanitize or disinfect a medical or dental appliance;
an ultrasonic cleaning device; the ultrasonic cleaning device preferably applies highfrequency sound waves, e.g., ultrasonic sound waves, and a liquid, in particular a cleaning solution, to sanitize or clean a medical or dental appliance;
a maintenance device; the maintenance device preferably applies at least one of a compressed gas, in particular air, or a lubricating agent to maintain a medical or dental appliance.

Depending on the type of medical or dental sanitizing and/ or maintenance device for sanitizing and/ or maintaining a medical or dental appliance the enclosure to accommodate the medical or dental appliance to be sanitized and/ or maintained may comprise e.g., a chamber or a tank. The enclosure is configured to accommodate or house the medical or dental appliance to be to be sanitized and/ or maintained and to keep the sanitizing and/ or maintaining agent. The enclosure may have an opening to insert and remove the medical or dental appliance. The enclosure may have a door or lid to close the opening.

Depending on the type of medical or dental sanitizing and/ or maintenance device for sanitizing and/ or maintaining a medical or dental appliance the feeding device for feeding a sanitizing and/ or maintaining agent into the enclosure preferably comprises at least one of: a line, a tube, a pump, a valve, a connector, a light guide, a bar to transmit vibrational or ultrasonic energy, a sonotrode, and similar components. Various sources for a sanitizing and/ or maintaining agent, e.g., a steam generator, a container for a liquid or a detergent, a source for compressed gas (air), a radiation source, a source for vibrational energy, and similar sources preferably couple to the feeding device, so that the feeding device can convey the sanitizing and/ or maintaining agent to and/ or into the enclosure.

The control unit is configured to control the operation of the medical or dental sanitizing and/ or maintenance device. According to an embodiment it controls a sanitizing and/ or maintaining procedure or cycle of the medical or dental sanitizing and/ or maintenance device. According to an embodiment it controls an indicator device, e.g., a LED and/ or a display of the medical or dental sanitizing and/ or maintenance device. According to an embodiment the control unit comprises or communicatively connects to a user interface, so that preferably a user can control or operate the medical or dental sanitizing and/ or maintenance device or various operations as described in this document.

These elements of the medical or dental sanitizing and/ or maintenance device described in the foregoing, i.e., the enclosure, the feeding device, and the control unit, and modifications of these elements, depending on the type of medical or dental sanitizing and/ or maintenance device are well known to a skilled person.

The imaging device which is configured and arranged to capture one or more images of the medical or dental appliance to be sanitized and/ or maintained preferably comprises at least one of: a light-sensitive integrated circuit or sensor, a light guide, an optical element, e.g., a lens, a camera, in particular a video camera. According to an embodiment, the imaging device can be arranged inside the medical or dental sanitizing and/ or maintenance device, e.g., inside an outer housing or shell and/ or the enclosure. According to an embodiment, the imaging device can be arranged at the outer housing or at the opening of the enclosure. According to an embodiment, the imaging device can be arranged outside the medical or dental sanitizing and/ or maintenance device, for example above or below, left, or right of the medical or dental sanitizing and/ or maintenance device. According to an embodiment, the imaging device has a field of view, wherein the imaging device is arranged such, that the field of view extends towards or into the enclosure and/ or out of the enclosure.

According to the invention the medical or dental sanitizing and/ or maintenance device further comprises an artificial intelligence module that is communicatively coupled to the control unit. According to an embodiment the artificial intelligence module is a separate unit, remote from the control unit, whereby the artificial intelligence module can be arranged in the medical or dental sanitizing and/ or maintenance device or outside the medical or dental sanitizing and/ or maintenance device, e.g., in a remote server or the Cloud and connect to the medical or dental sanitizing and/ or maintenance via the Internet. Alternatively, the artificial intelligence module is configured as a part of the control unit and/ or the control unit comprises the artificial intelligence module. The control unit and the artificial intelligence module can be communicatively coupled to one another wirelessly and/ or through a wired connection. The artificial intelligence module may be communicatively coupled to more than one medical or dental sanitizing and/ or maintenance devices, in particular if the artificial intelligence module is implemented in a remote server or in the Cloud. Thus, in an advantageous manner, training and learning by the artificial intelligence module is improved, since the number and variety of data supplied to the artificial intelligence module is higher, if a greater number of sanitizing and/ or maintenance devices is communicatively coupled to the artificial intelligence module.

The artificial intelligence module is also communicatively coupled to the imaging device to receive the one or more images, e.g., a video stream, of the medical or dental appliance captured by the imaging device. The imaging device and the artificial intelligence module can be communicatively coupled to one another wirelessly and/ or through a wired connection.

According to the invention the artificial intelligence module is preferably implemented through and/ or comprises a trained or trainable neural network. Preferably, the artificial intelligence module or neural network is implemented through one or more computer-implemented algorithms which is/ are running on a processing unit. Preferably, the artificial intelligence module or neural network applies deep learning. As described in the foregoing, this processing unit can either be a part of the control unit or separate from it. According to an embodiment the basic architecture of the artificial intelligence module comprises at least one of: a Convolutional Neural Network (CNN), a Recurrent Neural Network (RNN) or Transformer architecture or similar Neural Networks or architectures. According to an embodiment the artificial intelligence module or neural network is configured to perform object detection receiving a bounding box, using, e.g., the 'You only look once' (YOLO) algorithm or a Faster Region-based Convolutional Neural Network (R-CNN). According to an embodiment the artificial intelligence module or neural network is configured to perform object segmentation receiving a segmentation mask, using, e.g., a Mask R-CNN or Mask2Former algorithm.

According to an embodiment the artificial intelligence module or neural network is trained to recognize the medical or dental appliance to be sanitized and/ or maintained based on the received one or more images, and configured to output data on the recognized medical or dental appliance to be sanitized and/ or maintained, in particular to the control unit and/ or to a database, so that the control unit controls a sanitizing and/ or maintaining procedure of the medical or dental sanitizing and/ or maintenance device based on the provided output data on the recognized medical or dental appliance. Recognition of the medical or dental appliance and/ or the output data comprise(s) at least one of: a type of a medical or dental appliance, e.g., a handpiece, an endoscope, a pair of scissors, a pair of tweezers, a drill, a reamer, a saw, a motor drive, etc.; a model series of a medical or dental appliance, e.g., contra-angled handpiece of model series "A" or model series "S"; exact identification of the medical or dental appliance based on its unique labeling, e.g., a ref.-number and/ or an code (QR-code, bar code) on the medical or dental appliance.

According to an embodiment the artificial intelligence module or neural network is trained to determine the soiling of the medical or dental appliance to be sanitized and/ or maintained based on the received one or more images, and configured to output data on the soiling of medical or dental appliance to be sanitized and/ or maintained, in particular to the control unit and/ or to a database, so that the control unit controls a sanitizing and/ or maintaining procedure of the medical or dental sanitizing and/ or maintenance device based on the provided output data on the soiling. In particular, the artificial intelligence module is trained to determine the soiling of the outer surface of the medical or dental appliance to be sanitized and/ or maintained. Determination of the soiling by the artificial intelligence module and/ or the database comprise(s) at least one of: the quantity of soiling; the type of soiling, e.g., blood or no blood; the position and/ or distribution of the soiling on the medical or dental appliance, e.g., even distribution or a higher quantity of soiling at a portion of the medical or dental appliance, e.g., at an end or in the center.

According to an embodiment and based on the received one or more images, the artificial intelligence module or neural network is trained to recognize the medical or dental appliance to be sanitized and/ or maintained and to determine the soiling of the medical or dental appliance to be sanitized and/ or maintained, in particular the soiling of the outer surface, and configured to output data on the recognized medical or dental appliance to be sanitized and/ or maintained and the soiling of the medical or dental appliance to be sanitized and/ or maintained, in particular to the control unit and/ or to a database, so that the control unit controls a sanitizing and/ or maintaining procedure of the medical or dental sanitizing and/ or maintenance device based on the provided output data on the recognized medical or dental appliance and on the output data on the soiling. The output data comprise the same information as described in the foregoing.

These embodiments have a plurality of advantageous effects. First, there is no variability of the detection space. Thus, the risk of capturing data from a medical or dental appliance not to be treated by the medical or dental sanitizing and/ or maintenance device is decreased. A medical or dental appliance not to be sanitized and/ or maintained may thus be placed close to the medical or dental sanitizing and/ or maintenance device, without the risk that it might be detected, if it is outside the field of view of the imaging device. In particular, if the imaging device is arranged such that the field of view extends towards or into the enclosure or is inside the enclosure, and/ or the imaging device is arranged in the enclosure, at the opening or door of the medical or dental sanitizing and/ or maintenance device, the probability of capturing images of the medical or dental appliance is greatly reduced. Correspondingly, a medical or dental appliance has to be placed deliberately in the camera's field of view to be registered or imaged.

Another advantageous effect of the present invention is that a current condition, in particular the soiling, of a medical or dental appliance to be sanitized and/ or maintained can be registered. The current condition may be a condition before the start of a sanitizing and/ or maintenance procedure by the medical or dental sanitizing and/ or maintenance device and/ or a condition during a sanitizing and/ or maintenance procedure.

Another advantageous effect of the present invention is that the RFID-tags on the medical or dental appliances can be omitted.

Another advantageous effect is that with the imaging device a plurality of detection tasks or steps can be performed by the imaging device, e.g., the recognition of a medical or dental appliance to be sanitized and/ or maintained and the soiling of this appliance. Preferably, these detection tasks can be performed by using the same images captured by the imaging device and/ or at the same time. Thus, in advantageous way, the number of sensors to realize these detection tasks as well as the time to perform these detection tasks is reduced.

Preferably, in a training process the artificial intelligence module or neural network is trained to recognize the medical or dental appliance to be sanitized and/ or maintained and/ or to determine the soiling of the medical or dental appliance to be sanitized and/ or maintained based on the received one or more images. Generally, the aim of the training process is to train a non-trained artificial intelligence module to develop a trained artificial intelligence module, that is capable of processing the input one or more images to recognize the medical or dental appliance and/ or its soiling, in particular the soiling of the outer surface of the medical or dental appliance.

Preferably, the training process comprises a "supervised training".

According to an embodiment a dataset comprising a plurality of images of medical or dental appliances (to be sanitized and/ or maintained in the future), in particular images with more than one of these medical or dental appliances, is created. This plurality of images is labeled and fed to the artificial intelligence module or neural network, so that the artificial intelligence module learns to distinguish and recognize the medical or dental appliances. Features that may be used by the artificial intelligence module to recognize the medical or dental appliances may comprise at least one of: semantic information about the medical or dental appliance, e.g., symbols, alphanumeric characters, codes (e.g., a QR-code, bar code, etc.), and similar semantic information; geometric information about the medical or dental appliance, e.g., the shape of a portion or component of a medical or dental appliance and/ or the shape of the entire medical or dental appliance; a dimension of a portion or component of a medical or dental appliance and/ or of the entire medical or dental appliance. Next, images with one and/ or more of these medical or dental appliances are fed to the trained artificial intelligence module to validate the quality of recognition, i.e., whether the trained artificial intelligence module is already capable of recognizing or identifying the medical or dental appliances, in particular as described before capable of recognizing the type of a medical or dental appliance and/ or a model series of a medical or dental appliance and/ or an exact identification of the medical or dental appliance. If the quality of recognition is not sufficient, further training with additional datasets as described in the foregoing is performed. If the quality of recognition is sufficient, the training process is stopped, and the trained artificial intelligence module or neural network can be implemented into a medical or dental sanitizing and/ or maintenance device.

According to an embodiment a dataset comprising a plurality of images of soiled medical or dental appliances (to be sanitized and/ or maintained in the future), in particular images with different types of soiling and/ or different conditions of the soiling and/ or different quantity of soiling, is created. Preferably, the images show medical or dental appliances having a soiled outer surface. The soiling may comprise the type of soiling, e.g., at least one of: blood; saliva; particles. The soiling may comprise the condition of the soling, e.g., at least one of: fresh soiling, dried soiling, caked-on soiling. The soiling may comprise different quantities of soiling of the outer surface of the medical or dental appliances, ranging from 0% soiling to 100% soiling; or categories of soiling e.g., no soiling, light soiling, medium soiling, heavy soiling. This plurality of images is labeled and fed to the artificial intelligence module, so that the artificial intelligence module learns to distinguish and determine the soiling. Next, images with one and/ or more soiled medical or dental appliances are fed to the trained artificial intelligence module to validatethe quality of determination, i.e., whether the trained artificial intelligence module is already capable of recognizing or determine the soiling, in particular as described before capable of recognizing the type of soiling and/ or the condition of the soiling and/ or the quantities of soiling, in particular the soiling of the outer surface. If the quality of determination is not sufficient, further training with additional datasets as described in the foregoing is performed. If the quality of recognition is sufficient, the training process is stopped, and the trained artificial intelligence module or neural network can be implemented into a medical or dental sanitizing and/ or maintenance device.

According to an embodiment the two training processes described in the foregoing can be performed by the (one) artificial intelligence module, so that the artificial intelligence module is trained to recognizing the medical or dental appliances to be sanitized and/ or maintained and to determine the soiling of the medical or dental appliances, in particular of their outer surfaces. The training process is performed in the same way as described above, so that finally the artificial intelligence module that can recognizing the medical or dental appliances determine the soiling can be implemented into a medical or dental sanitizing and/ or maintenance device.

Particularly preferred, also datasets with images comprising medical or dental objects which must not be sanitized and/ or maintained are created, so that the artificial intelligence module or neural network is in addition trained to recognize these objects, e.g., gloves, single-use objects, e.g., a single-use syringe or a single-use tube, bandaging material, and similar objects. The training process is the same as described in the previous paragraphs. The aim of training the artificial intelligence module to recognize objects that must not be sanitized and/ or maintained is that, based on the output data on these medical or dental appliances not to be sanitized and/ or maintained by the artificial intelligence module, in particular to the control unit and/ or to a database, the control unit is configured not to start a sanitizing and/ or maintaining procedure and/ or to issue an information or alarm. Accordingly, based on the received one or more images the artificial intelligence module of the medical or dental sanitizing and/ or maintenance device can recognize a medical or dental appliance which is not configured to or must not be sanitized and/ or maintained by the medical or dental sanitizing and/ or maintenance device. Thus, in an advantageous manner, sanitizing and/ or maintaining of a medical or dental appliance not to be sanitized and/ or maintained and resulting consequences, e.g., soiling and/ or damage of the medical or dental sanitizing and/ or maintenance device, can be avoided.

According to an embodiment, the trained artificial intelligence module is continued to be trained after being implemented in a medical or dental sanitizing and/ or maintenance device, e.g., in view of medical or dental appliances and/ or soiling which have not been trained before and/ or dental appliances that become newly available on the market and/ or to improve the performance of the trained artificial intelligence. Thus, in an advantageous manner, the trained artificial intelligence module is improved, kept up to date and is not limited to the medical or dental appliances it was initially trained. Preferably, a user interface, in particular the user interface coupled to the control unit, is provided on the medical or dental sanitizing and/ or maintenance device, for the continuous training of the medical or dental sanitizing and/ or maintenance device. Preferably, the user interface is communicatively coupled to the artificial intelligence module to train the artificial intelligence module. Preferably, the user interface comprises input elements, e.g., a keyboard and/ or a microphone, to label newly added medical or dental appliances or soiling. Preferably, the medical or dental sanitizing and/ or maintenance device comprises an imaging device or a data transmission device, e.g., a connection port to the internet or to a data carrier, to provide an image of a medical or dental appliance or soiling to be added to the artificial intelligence module. The imaging device may be the same that is used to provide one or more images to the trained artificial intelligence module when the medical or dental sanitizing and/ or maintenance device is operated to sanitize and/ or maintain a medical or dental appliance or it may be a different one.

Preferably, the artificial intelligence module or neural network is trained and/ or configured to recognize the medical or dental appliance to be sanitized and/ or maintained and/ or to determine the soiling of the medical or dental appliance to be sanitized and/ or maintained, in particular the soiling of the outer surface, when the medical or dental appliance is arranged in a pouch or on a tray or similar supporting structure of a medical or dental sanitizing and/ or maintenance device having at least a transparent portion. The transparent portion may comprise, e.g., a transparent foil as part of a pouch or a transparent plastic material as part of a tray. In particular, the imaging device is configured and arranged to capture one or more images of the medical or dental appliance to be sanitized and/ or maintained through said transparent portion and to transmit the one or more images to the artificial intelligence module, which recognizes the medical or dental appliance and/ or the soiling in the same way as described in the foregoing. Also, the training of the artificial intelligence module is done in the same way as described in the foregoing, wherein the plurality of images of medical or dental appliances (to be sanitized and/ or maintained in the future) labeled and fed to the artificial intelligence module comprise at least some images with a medical or dental appliance in a pouch or on a tray or similar supporting structure.

According to an embodiment the medical or dental sanitizing and/ or maintenance device further comprises a database which, based on the output data on the recognized medical or dental appliance, provides appliance data on the recognized medical or dental appliance to be sanitized and/ or maintained to the control unit. Alternatively, or in addition, the medical or dental sanitizing and/ or maintenance device comprises a database which, based on the output data on the soiling, provides sanitizing and/ or maintenance information to the control unit. Obviously, the medical or dental sanitizing and/ or maintenance device may comprise a single database which provides appliance data and sanitizing and/ or maintenance information. Preferably, the database is stored on and/ or comprises a (non-transitory) storage medium or memory. The database or storage medium can be a separate unit, remote from the control unit and/ or the artificial intelligence module, wherein the storage medium can be arranged in the medical or dental sanitizing and/ or maintenance device or outside the medical or dental sanitizing and/ or maintenance device, e.g., in a remote server or the Cloud and connect to the medical or dental sanitizing and/ or maintenance via the Internet. Alternatively, the database or storage medium is configured as a part of the control unit and/ or of the artificial intelligence module. The control unit and the database as well as the artificial intelligence module and the database can be communicatively coupled to one another wirelessly and/ or through a wired connection. The database may be communicatively coupled to more than one medical or dental sanitizing and/ or maintenance device, in particular if implemented in a remote server or in the Cloud. Thus, in an advantageous manner, updating a plurality of medical or dental sanitizing and/ or maintenance devices connected to one database with a new medical or dental appliance can be done quicker.

In particular, the appliance data on the recognized medical or dental appliance and/ or the sanitizing and/ or maintenance information provided by the database may be used - either alone or together with the output data of the artificial intelligence module - to control a sanitizing and/ or maintaining procedure of the medical or dental sanitizing and/ or maintenance device by the control unit, as described in more detail below. Thus, in an advantageous manner, a sanitizing and/ or maintaining procedure can be adapted to the medical or dental appliance to be sanitized and/ or maintained and/ or to the soiling.

Preferably, the database stores appliance data on medical or dental appliances that can be recognized by the artificial intelligence module. Preferably, the database is configured to receive the output data on the recognized medical or dental appliance to be sanitized and/ or maintained from the artificial intelligence module. Preferably, based on the received output data, the database is configured to retrieve the stored appliance data on the recognized medical or dental appliance and to transmit it to the control unit. Thus, in an advantageous manner, a sanitizing and/ or maintaining procedure can be adapted to the medical or dental appliance to be sanitized and/ or maintained.

Preferably, the database stores sanitizing and/ or maintenance information about soiling that can be recognized by the artificial intelligence module. Preferably, the database is configured to receive the output data on the soiling from the artificial intelligence module. Preferably, based on the received output data, the database is configured to retrieve the stored sanitizing and/ or maintenance information and to transmit it to the control unit. Thus, in an advantageous manner, a sanitizing and/ or maintaining procedure can be adapted to the soiling of the medical or dental appliance to be sanitized and/ or maintained.

Preferably, the database provides appliance data on technical characteristics of the medical or dental appliance to be sanitized and/ or maintained. The technical characteristics may comprise for example at least one of: whether the medical or dental appliance has an internal hollow space; whether the medical or dental appliance has an electronic component; the material of the outer surface or sleeve of the medical or dental appliance; whether the medical or dental appliance has rotatable elements; and similar technical characteristics. Thus, in an advantageous manner, a sanitizing and/ or maintaining procedure performed by the medical or dental sanitizing and/ or maintenance device can be adapted to the technical characteristics of the medical or dental appliance to be sanitized and/ or maintained.

Preferably, the database provides appliance data or sanitizing and/ or maintenance information about the kind of sanitizing and/ or maintenance which may be applied and/ or which must not be applied to a medical or dental appliance. These data may comprise e.g., at least one of that a medical or dental appliance: may be sterilized; must not be sterilized; may be exposed to steam; must not be exposed to steam; may be disinfected with a detergent; must not be disinfected with a detergent; may be washed in a TWD; must not be washed in a TWD; may be lubricated; must not be lubricated; may be soaked in a cleaning liquid; must not be soaked in a cleaning liquid; and similar data. These data may also comprise at least one of: a sanitizing and/ or maintaining cycle to be applied to a medical or dental appliance; duration of a sanitizing and/ or maintaining procedure; duration of a step of a sanitizing and/ or maintaining procedure; determination of an operating parameter of the sanitizing and/ or maintaining procedure; and similar data. Thus, in an advantageous manner, only a suitable sanitizing and/ or maintaining procedure is applied to the medical or dental appliance to be sanitized and/ or maintained.

Preferably, the database provides sanitizing and/ or maintenance information to the control unit which comprises e.g., at least one of: a sanitizing and/ or maintaining cycle to be performed; omission of a step of a predefined sanitizing and/ or maintaining cycle; determination of a duration of the sanitizing and/ or maintaining procedure; determination of an operating parameter of the sanitizing and/ or maintaining procedure; determination of a duration of a step of the sanitizing and/ or maintaining procedure; determination of a temperature in a sanitizing procedure; determination of a pressure in a sanitizing procedure; determination of a power input in a sanitizing procedure. Thus, in an advantageous manner, a sanitizing and/ or maintaining procedure can be adapted to the soiling determined by the artificial intelligence module.

Preferably, the database provides sanitizing and/ or maintenance information to the control unit which comprises e.g., at least one of: determination of a sanitizing and/ or maintaining agent; determination of a concentration of a sanitizing and/ or maintaining agent; determination of an exposure time of a sanitizing and/ or maintaining agent. Thus, in an advantageous manner, a sanitizing and/ or maintaining agent and/ or its application can be adapted to the soiling determined by the artificial intelligence module.

As already described in the foregoing, the control unit is configured to control a sanitizing and/ or maintaining procedure of the medical or dental sanitizing and/ or maintenance device based on the output data of the artificial intelligence module and/ or the appliance data and/ or the sanitizing and/ or maintenance information of the database. According to an embodiment, controlling the sanitizing and/ or maintaining procedure by the control unit may comprise at least one of: determination of a sanitizing and/ or maintaining cycle; omission of a step of a predefined sanitizing and/ or maintaining cycle; determination of a duration of the sanitizing and/ or maintaining procedure; determination of an operating parameter of the sanitizing and/ or maintaining procedure; determination of a duration of a step of the sanitizing and/ or maintaining procedure; determination of a temperature in a sanitizing procedure; determination of a pressure in a sanitizing procedure; determination of a power input in a sanitizing procedure; and similar specifications. Thus, in an advantageous manner, only a suitable sanitizing and/ or maintaining procedure, duration of such a procedure, etc. is applied to the medical or dental appliance to be sanitized and/ or maintained.

In addition or alternatively, controlling the sanitizing and/ or maintaining procedure by the control unit may comprise at least one of: determination of a sanitizing and/ or maintaining agent; determination of a concentration of a sanitizing and/ or maintaining agent; determination of an exposure time of a sanitizing and/ or maintaining agent; and similar specifications. Thus, in an advantageous manner, only a suitable sanitizing and/ or maintaining agent, concentration and/ or exposure time is applied to the medical or dental appliance to be sanitized and/ or maintained.

Preferably, the medical or dental sanitizing and/ or maintenance device comprises a display which is configured to:
- visually display information about the medical or dental appliance to be sanitized and/ or maintained, based on the output data provided by the artificial intelligence module and/ or on the appliance data provided by the database, and/ or
- visually display information about the sanitizing and/ or maintaining procedure to be performed by the medical or dental sanitizing and/ or maintenance device, based on the output data provided by the artificial intelligence module and/ or appliance data and/ or sanitizing and/ or maintenance information provided by the database, and/ or
- visually display information about the soiling (of the medical or dental sanitizing and/ or maintenance device), based on the output data provided by the artificial intelligence module and/ or sanitizing and/ or maintenance information provided by the database.

Thus, in an advantageous manner, a user can check the displayed information and can, if necessary, alter or correct this information. Preferably, a user interface, in particular the user interface coupled to the control unit, is provided on the medical or dental sanitizing and/ or maintenance device, for altering or correcting the information.

Preferably, the medical or dental sanitizing and/ or maintenance device is configured to issue an error signal if two medical or dental appliances arranged to be sanitized and/ or maintained in a shared sanitizing and/ or maintaining procedure require a different sanitizing and/ or maintaining procedure, based on the output data provided by the artificial intelligence module on these two medical or dental appliances and/ or on the soiling (of these two medical or dental appliances), and/ or based on the appliance data and/ or sanitizing and/ or maintenance information provided by the database. The different sanitizing and/ or maintaining procedure may comprise e.g., a different sanitizing and/ or maintaining cycle and/ or a different sanitizing and/ or maintaining parameter (e.g., temperature, pressure, etc.) and/ or a different sanitizing and/ or maintaining agent and/ or similar specifications. For example, the medical or dental sanitizing and/ or maintenance device is configured to issue an error signal if a first medical or dental appliance requires autoclaving, while a second, different medical or dental appliance must not be exposed to autoclaving. For example, the medical or dental sanitizing and/ or maintenance device is configured to issue an error signal if a first medical or dental appliance requires lubrication, while a second, different medical or dental appliance needs no lubrications, since it has no rotating elements. Obviously, a skilled person can think of many other examples, i.e., non-suitable arrangements of medical or dental appliances, which might cause the medical or dental sanitizing and/ or maintenance device to issue an error signal.

In particular, the control unit is configured to compare the received output data and/ or appliance data and/ or sanitizing and/ or maintenance information and - if, based on the received data, there is a discrepancy between sanitizing and/ or maintaining requirements or sanitizing and/ or maintaining procedures of two medical or dental appliances - to activate a warning device to issue an error signal. The error signal can comprise a visual, acoustic and/ or haptic signal. Preferably, the error signal comprises the visual indication of at least one medical or dental appliance which requires a different sanitizing and/ or maintaining procedure. Thus, in an advantageous manner, problems which might arise if a medical or dental appliance is sanitized and/ or maintained in an unsuitable sanitizing and/ or maintaining procedure, e.g., damage of this medical or dental appliance and/ or of the medical or dental sanitizing and/ or maintenance device, can be avoided.

According to an embodiment, a computer-implemented method, in particular a computer-implemented method for sanitizing and/ or maintaining a medical or dental appliance, is defined in that: a trained artificial intelligence module receives one or more images of a medical or dental appliance to be sanitized and/ or maintained captured by an imaging device of a medical or dental sanitizing and/ or maintenance device, in particular a medical or dental sanitizing and/ or maintenance device as described in this document. The trained artificial intelligence module recognizes the medical or dental appliance to be sanitized and/ or maintained based on the received one or more images, and outputs data on the recognized medical or dental appliance to be sanitized and/ or maintained, in particular to a control unit and/ or database of the medical or dental sanitizing and/ or maintenance device. The control unit controls a sanitizing and/ or maintaining procedure of the medical or dental sanitizing and/ or maintenance device based on the provided output data.

Preferably, based on the output data, the database provides appliance data on the recognized medical or dental appliance to be sanitized and/ or maintained to the control unit as described in the foregoing. Preferably, the control unit is configured to control a sanitizing and/ or maintaining procedure of the medical or dental sanitizing and/ or maintenance device based on the output data of the artificial intelligence module and/ or the appliance data of the database as described in the foregoing.

According to an embodiment, a computer-implemented method, in particular a computer-implemented method for sanitizing and/ or maintaining a medical or dental appliance, is defined in that: a trained artificial intelligence module receives one or more images of a medical or dental appliance to be sanitized and/ or maintained captured by an imaging device of a medical or dental sanitizing and/ or maintenance device, in particular a medical or dental sanitizing and/ or maintenance device as described in this document. The trained artificial intelligence module determines the soiling of the medical or dental appliance to be sanitized and/ or maintained based on the received one or more images, and outputs data on the soiling, in particular to a control unit and/ or database of the medical or dental sanitizing and/ or maintenance device. The control unit controls a sanitizing and/ or maintaining procedure of the medical or dental sanitizing and/ or maintenance device based on the provided output data on the soiling.

Preferably, based on the output data on the soiling, the database provides sanitizing and/ or maintenance information to the control unit as described in the foregoing. Preferably, the control unit is configured to control a sanitizing and/ or maintaining procedure of the medical or dental sanitizing and/ or maintenance device based on the output data of the artificial intelligence module and/ or the sanitizing and/ or maintenance information of the database as described in the foregoing.

Preferably, the computer-implemented methods comprise one or more of the steps, devices and/ or features described in the foregoing.

Preferably, the trained artificial intelligence module is implemented through and/ or comprises a trained or trainable neural network as described in the foregoing. The training of the artificial intelligence module or neural network is done as described in the foregoing.

According to an embodiment, a computer program product or computer-readable (non-transitory) storage medium comprises instructions which, when executed by a computer, cause the computer to execute one of the or both computer-implemented methods, in particular the computer-implemented method for sanitizing and/ or maintaining a medical or dental appliance, as described in the foregoing.

These and other preferred embodiments will be described below with reference to the following drawings:
Figure 1 shows a schematic diagram of a first embodiment of a medical or dental sanitizing and/ or maintenance device for sanitizing and/ or maintaining a medical or dental appliance having an artificial intelligence module that is trained to output data, so that the control unit controls a sanitizing and/ or maintaining procedure of the medical or dental sanitizing and/ or maintenance device based on the provided output data.
Figure 2 shows a schematic diagram of a second embodiment of a medical or dental sanitizing and/ or maintenance device for sanitizing and/ or maintaining a medical or dental appliance having an artificial intelligence module that is trained to output data, so that the control unit controls a sanitizing and/ or maintaining procedure of the medical or dental sanitizing and/ or maintenance device based on the provided output data.
Figure 3 shows steps of a computer-implemented method for sanitizing and/ or maintaining a medical or dental appliance using a trained artificial intelligence module, so that a control unit of a medical or dental sanitizing and/ or maintenance device controls a sanitizing and/ or maintaining procedure.
Figure 4 shows a schematic diagram of an embodiment of a medical or dental sanitizing and/ or maintenance device for sanitizing and/ or maintaining a medical or dental appliance according to Figures 1 or 2 in the form of a sterilizer or autoclave.
Figure 5 shows a schematic diagram of an embodiment of a medical or dental sanitizing and/ or maintenance device for sanitizing and/ or maintaining a medical or dental appliance according to Figures 1 or 2 in the form of a thermal cleaning and disinfecting device or thermal washer disinfector.
Figure 6 shows a schematic diagram of an embodiment of a medical or dental sanitizing and/ or maintenance device for sanitizing and/ or maintaining a medical or dental appliance according to Figures 1 or 2 in the form of an ultrasonic cleaning device.
Figure 7 shows a schematic diagram of an embodiment of a medical or dental sanitizing and/ or maintenance device for sanitizing and/ or maintaining a medical or dental appliance according to Figures 1 or 2 in the form of a maintenance device.

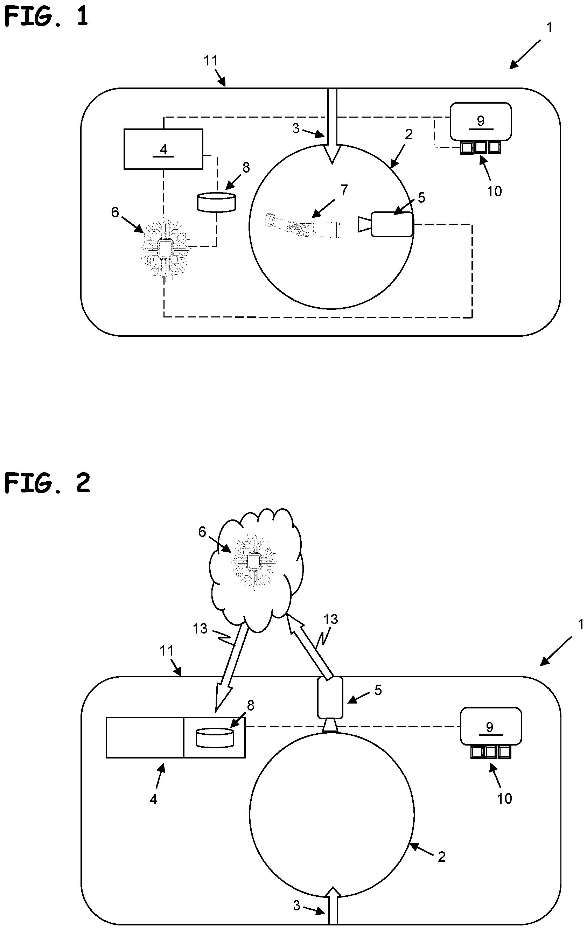

Figures 1 and 2 show a schematic diagram of a medical or dental sanitizing and/ or maintenance device 1 for sanitizing and/ or maintaining a medical or dental appliance 7. The medical or dental appliance 7 shown in Figure 1 comprises a contra-angle handpiece, which is to be understood as a symbolic placeholder for any medical or dental appliance that needs sanitizing and/ or maintenance. The medical or dental sanitizing and/ or maintenance device 1 comprises an outer housing or shell 11 which holds amongst others an enclosure 2 to accommodate the medical or dental appliance 7 to be sanitized and/ or maintained, at least parts of a feeding device 3 for feeding a sanitizing and/ or maintaining agent into the enclosure 2 to sanitize or maintain the medical or dental appliance, and a control unit 4 to control operation of the medical or dental sanitizing and/ or maintenance device 1.

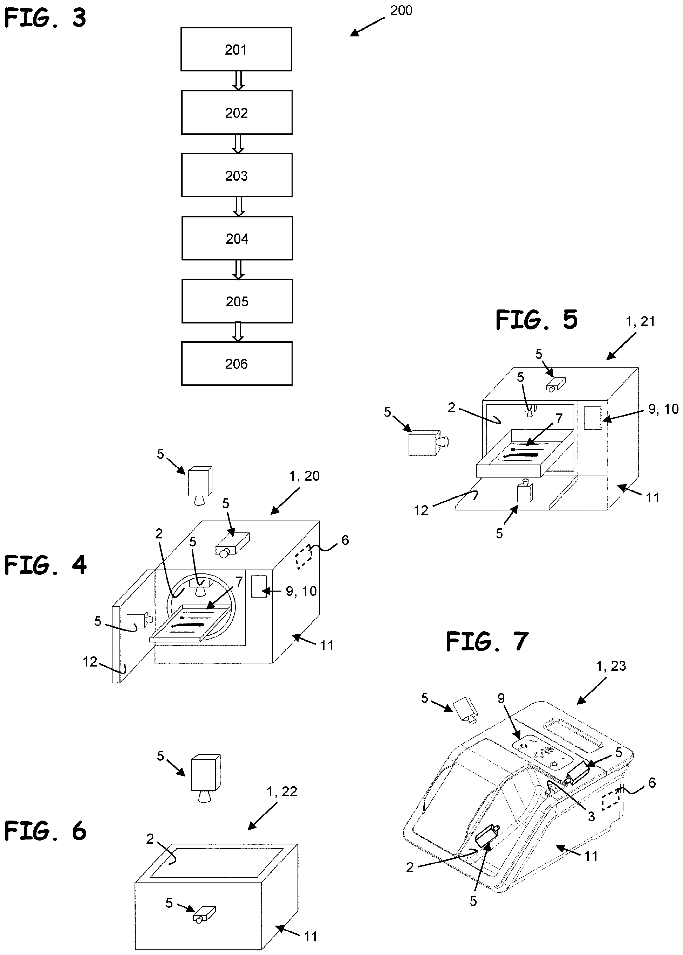

The enclosure 2 to accommodate the medical or dental appliance 7 to be sanitized and/ or maintained may comprise a cylindric chamber, e.g., if the medical or dental sanitizing and/ or maintenance device 1 comprises a sterilizer or autoclave (see Fig. 4), or an substantially cuboidal tank, e.g., if the medical or dental sanitizing and/ or maintenance device 1 is a TWD (see Fig. 5), an ultrasonic cleaning device (see Fig. 5) or a maintenance device (see Fig. 7). Preferably, the enclosure 2 has an opening through which a portion of the feeding device 3 extends and/ or a sanitizing and/ or maintaining agent is fed into the interior of the enclosure 2.

The control unit 4 is configured to control the operation of the medical or dental sanitizing and/ or maintenance device 1, in particular one or more sanitizing and/ or maintaining procedures or cycles of the medical or dental sanitizing and/ or maintenance device. The control unit 4 also communicatively connects and controls an indicator device, e.g., a LED and/ or a display 9, of the medical or dental sanitizing and/ or maintenance device 1. Further, the control unit 4 communicatively connects to a user interface 10, so that a user can control or operate the medical or dental sanitizing and/ or maintenance device 1 or various operations as described in this document. The communicative connections of the control unit 4 are shown by dashed lines in Fig. 1.

The medical or dental sanitizing and/ or maintenance device 1 further comprises an imaging device 5 which is configured and arranged to capture one or more images of the medical or dental appliance 7 to be sanitized and/ or maintained. Preferably, the imaging device 5 comprises a digital camera. The imaging device 5 is communicatively connected to an artificial intelligence module 6, preferably also to the control unit 4 and/ or a database 8, see Fig. 1.

The artificial intelligence module 6 is communicatively coupled to the control unit 4, and to the imaging device 5 to receive the one or more images of the medical or dental appliance 7 to be sanitized and/ or maintained captured by the imaging device 5. The artificial intelligence module 6 is trained to recognize the medical or dental appliance 7 to be sanitized and/ or maintained based on the received one or more images, and configured to output data on the recognized medical or dental appliance 7 to be sanitized and/ or maintained. The output data on the recognized medical or dental appliance 7 are transmitted to the control unit directly and/ or via the database 8, so that the control unit 4 controls a sanitizing and/ or maintaining procedure of the medical or dental sanitizing and/ or maintenance device 1 based on the provided data on the recognize the medical or dental appliance 7 to be sanitized and/ or maintained.

Alternatively, or in addition, the artificial intelligence module 6 is trained to determine the soiling of the medical or dental appliance 7 to be sanitized and/ or maintained based on the received one or more images, and configured to output data on the soiling of medical or dental appliance 7 to be sanitized and/ or maintained. The output data on the recognized medical or dental appliance 7 are transmitted to the control unit directly and/ or via the database 8, so that the control unit 4 controls a sanitizing and/ or maintaining procedure of the medical or dental sanitizing and/ or maintenance device 1 based on the provided output data on the soiling.

Based on the output data on the recognized medical or dental appliance 7, the database 8 is configured to provide appliance data on the recognized medical or dental appliance 7 to be sanitized and/ or maintained to the control unit 4. Alternatively, or in addition, the database 8 is configured to provide sanitizing and/ or maintenance information to the control unit 4. In particular, the appliance data on the recognized medical or dental appliance 7 and/ or the sanitizing and/ or maintenance information provided by the database 8 may be used - either alone or together with the output data of the artificial intelligence module 6 - to control a sanitizing and/ or maintaining procedure of the medical or dental sanitizing and/ or maintenance device 1 by the control unit 4.

In Figure 1 the artificial intelligence module 6 and the database 8 are arranged in the medical or dental sanitizing and/ or maintenance device 1, in particular in the interior of outer housing 11. The artificial intelligence module 6 and the database 8 are discrete components of the medical or dental sanitizing and/ or maintenance device 1, in particular separate of control unit 4. The artificial intelligence module 6, the database 8 and the control unit 4 connect with one another by wired or wireless communication appliances.

According to Figure 2, the artificial intelligence module 6 is disposed outside and/ or remote of the medical or dental sanitizing and/ or maintenance device 1, in particular of outer housing 11. Preferably, the artificial intelligence module 6 is arranged in the Cloud and communicatively connected to the imaging device 5 and the control unit 4, preferably also to the database 8, via the Internet (see arrows 13). Further, the database 8 is part of or comprised by control unit 4.

Alternative arrangements of the artificial intelligence module 6 and the database 8 are also conceivable. Any of the three possible arrangements - separate of control unit 4 and in the interior of outer housing 11; comprised by the control unit 4 and in the interior of outer housing 11; outside the medical or dental sanitizing and/ or maintenance device 1 - may apply to at least one of the artificial intelligence module 6 and database 8. Further, any combination of one the three arrangements of the artificial intelligence module 6 with one of one the three arrangements of the database 8 can be established. In particular the arrangement of at least one of the artificial intelligence module 6 and database 8 remote from the medical or dental sanitizing and/ or maintenance device 1, preferably in the Cloud, is advantageous, since a plurality of medical or dental sanitizing and/ or maintenance devices 1 can thus easily connect to the artificial intelligence module 6 and/ or database 8. In particular, when the trained artificial intelligence module 6 is continued to be trained after being implemented in a medical or dental sanitizing and/ or maintenance device 1, a higher number of and variety of data is supplied to the artificial intelligence module 6.

The medical or dental sanitizing and/ or maintenance device 1 further comprises a display 9. The display 9 is configured to visually display various information as described in the foregoing, e.g., based on the output data provided by the artificial intelligence module 6 and/ or appliance data provided by the database 8, information about the medical or dental appliance 7 to be sanitized and/ or maintained, and/ or on the sanitizing and/ or maintaining procedure to be performed by the medical or dental sanitizing and/ or maintenance device 1, and/ or information about the soiling, and/ or an error signal if two medical or dental appliances arranged to be sanitized and/ or maintained in a shared sanitizing and/ or maintaining procedure require a different sanitizing and/ or maintaining procedure.

The medical or dental sanitizing and/ or maintenance device 1 comprises a user interface 10 which, amongst others, is communicatively coupled to the artificial intelligence module 6 to train the artificial intelligence module 6, in particular if training after implementation of the artificial intelligence module 6 in a medical or dental sanitizing and/ or maintenance device 1 is continued. The user can thus teach the artificial intelligence module 6 a new medical or dental appliance 7 or soiling.

Figure 3 shows steps of a computer-implemented method 200 for sanitizing and/ or maintaining a medical or dental appliance 7 using a trained artificial intelligence module 6, so that a control unit 4 of a medical or dental sanitizing and/ or maintenance device 1 controls a sanitizing and/ or maintaining procedure: in step 201 the imaging device 5 captures one or more images of a medical or dental appliance 7 to be sanitized and/ or maintained. The one or more images are transmitted to the trained artificial intelligence module 6, which receives these one or more images in step 202. In step 203 the trained artificial intelligence module 6 recognizes the medical or dental appliance 7 to be sanitized and/ or maintained based on the received one or more images, and outputs in step 204 data on the recognized medical or dental appliance 7 to be sanitized and/ or maintained. These output data are received by the database 8, which provides in step 205 based on these received data appliance data on the recognized medical or dental appliance 7 to be sanitized and/ or maintained to the control unit 4. Finally, in step 206, the control unit 4 controls a sanitizing and/ or maintaining procedure of the medical or dental sanitizing and/ or maintenance device 1 referring to the appliance data which are based on the output data by the artificial intelligence module 6.

Alternatively, or in addition, in step 203 the trained artificial intelligence module 6 determines the soiling of the medical or dental appliance 7 to be sanitized and/ or maintained based on the received one or more images, and outputs in step 204 data on the soiling of the medical or dental appliance 7 to be sanitized and/ or maintained. These output data are received by the database 8, which provides in step 205 based on these received data sanitizing and/ or maintenance information to the control unit 4. Finally, in step 206, the control unit 4 controls a sanitizing and/ or maintaining procedure of the medical or dental sanitizing and/ or maintenance device 1 referring to the sanitizing and/ or maintenance information which are based on the output data by the artificial intelligence module 6.

Figures 4 - 7 show concrete embodiments of a medical or dental sanitizing and/ or maintenance device 1 described in the forgoing, in particular in Figures 1 and 2. Accordingly (although possibly not explicitly depicted in the Figures 4 - 7), each of the devices shown in Figures 4 - 7, comprises at least the enclosure 2 to accommodate the medical or dental appliance 7 to be sanitized and/ or maintained, the feeding device 3 for feeding a sanitizing and/ or maintaining agent into the enclosure 2, the control unit 4 to control operation of the medical or dental sanitizing and/ or maintenance device 1, the imaging device 5 which is configured and arranged to capture one or more images of the medical or dental appliance 7 to be sanitized and/ or maintained, and the artificial intelligence module 6 which is trained to recognize the medical or dental appliance 7 to be sanitized and/ or maintained and/ or the soiling of the medical or dental appliance 7 to be sanitized and/ or maintained based on the received one or more images. Depending on the type of the medical or dental sanitizing and/ or maintenance device 1, the embodiments of Figures 4 - 7 may additionally comprise one or more of the components 8 - 11.

Further, each of Figures 4 - 7 shows a plurality of imaging devices or cameras 5: while it may be possible that a medical or dental sanitizing and/ or maintenance device 1 has more than one imaging device 5, usually each medical or dental sanitizing and/ or maintenance device 1 comprises only one (a single) imaging device 5. Accordingly, each imaging device 5 shown in one of the Figures 4 - 7 shows a possible position of an imaging device 5 in relation to the respective medical or dental sanitizing and/ or maintenance device 1.

The medical or dental sanitizing and/ or maintenance devices 1 in Figure 4 is a sterilizer or autoclave 20, in particular a steam autoclave. As shown, autoclave 20 has an imaging device 5, which may be positioned inside the enclosure or chamber 2, on a door 12 to close the chamber 2, outside of the autoclave 20, e.g., disposed on the outer housing 11, or remote from the outer housing 11.

The medical or dental sanitizing and/ or maintenance devices 1 in Figure 5 is a thermal washer disinfector (TWD) 21. As shown, TWD 21 has an imaging device 5, which may be positioned inside the enclosure or chamber 2, on a door 12 to close the chamber 2, outside of the TWD 21, e.g., disposed on the outer housing 11, or remote from the outer housing 11.

The medical or dental sanitizing and/ or maintenance devices 1 in Figure 6 is an ultrasonic cleaning device 22. As shown, ultrasonic cleaning device 22 has an imaging device 5, which may be positioned outside, e.g., disposed on the outer housing 11, or remote from the outer housing 11.

The medical or dental sanitizing and/ or maintenance devices 1 in Figure 7 is a maintenance device 23. As shown, the maintenance device 23 has an imaging device 5, which may be positioned inside the enclosure or chamber 2 or outside of the maintenance device 23, e.g., disposed on the outer housing 11, or remote from the outer housing 11.

As an example, autoclave 20 and maintenance device 23 each have a trained artificial intelligence module 6 arranged inside the outer housing 11, while the trained artificial intelligence module 6 of TWD 21 and ultrasonic cleaning device 22 is a remote module which is arranged in the Cloud, as shown in Figure 2. Of course, according to other embodiments not shown, the trained artificial intelligence module 6 of autoclave 20 and maintenance device 23 can be remote, arranged in the Cloud, while the trained artificial intelligence module 6 of TWD 21 and ultrasonic cleaning device 22 can be arranged in the respective outer housing 11.

The embodiments described and shown, in particular, serve to depict the invention. The characteristics, disclosed in an embodiment, are therefore not limited to that embodiment, but can rather be combined individually or together with one or more characteristics of one of the other embodiments.

## Claims

1. A medical or dental sanitizing and/ or maintenance device (1) for sanitizing and/ or maintaining a medical or dental appliance (7), comprising:
an enclosure (2) to accommodate the medical or dental appliance (7) to be sanitized and/ or maintained,
a feeding device (3) for feeding a sanitizing and/ or maintaining agent into the enclosure (2) to sanitize or maintain the medical or dental appliance,
a control unit (4) to control operation of the medical or dental sanitizing and/ or maintenance device (1), **characterized by**
an imaging device (5) which is configured and arranged to capture one or more images of the medical or dental appliance (7) to be sanitized and/ or maintained, and
an artificial intelligence module (6) which is:
communicatively coupled to the control unit (4), and to the imaging device (5) to receive the one or more images of the medical or dental appliance (7) to be sanitized and/ or maintained captured by the imaging device (5),
trained to recognize the medical or dental appliance (7) to be sanitized and/ or maintained based on the received one or more images, and
configured to output data on the recognized medical or dental appliance (7) to be sanitized and/ or maintained, so that the control unit (4) controls a sanitizing and/ or
maintaining procedure of the medical or dental sanitizing and/ or maintenance device (1) based on the provided output data.

2. The medical or dental sanitizing and/ or maintenance device (1) according to Claim 1, **characterized in that**
it further comprises a database (8) which, based on the output data, provides appliance data on the recognized medical or dental appliance (7) to be sanitized and/ or maintained to the control unit (4).

3. The medical or dental sanitizing and/ or maintenance device (1) according to Claim 2, **characterized in that**
the database (8) provides appliance data on technical characteristics of the medical or dental appliance (7) to be sanitized and/ or maintained.

4. The medical or dental sanitizing and/ or maintenance device (1) according to Claim 2 or 3, **characterized in that**
the database (8) provides appliance data on the kind of sanitizing and/ or maintenance which may be applied and/ or which must not be applied to a medical or dental appliance (7) to be sanitized and/ or maintained.

5. The medical or dental sanitizing and/ or maintenance device (1) according to any one of the preceding Claims, **characterized in that**
based on the received one or more images the artificial intelligence module (6) is further trained to provide data on the soiling of the medical or dental appliance.

6. The medical or dental sanitizing and/ or maintenance device (1) according to any one of the preceding Claims, **characterized in that**
based on the received one or more images the artificial intelligence module (6) is further trained to recognize a medical or dental appliance which is not configured to be sanitized and/ or maintained by the medical or dental sanitizing and/ or maintenance device (1).

7. The medical or dental sanitizing and/ or maintenance device (1) according to any one of the preceding Claims, **characterized in that**
controlling the sanitizing and/ or maintaining procedure by the control unit (4) comprises at least one of: determination of a sanitizing and/ or maintaining cycle; omission of a step of a predefined sanitizing and/ or maintaining cycle; determination of a duration of the sanitizing and/ or maintaining procedure; determination of an operating parameter of the sanitizing and/ or maintaining procedure; determination of a duration of a step of the sanitizing and/ or maintaining procedure; determination of a temperature in a sanitizing procedure; determination of a pressure in a sanitizing procedure; determination of a power input in a sanitizing procedure.

8. The medical or dental sanitizing and/ or maintenance device (1) according to any one of the preceding Claims, **characterized in that**
controlling the sanitizing and/ or maintaining procedure by the control unit (4) comprises at least one of: determination of a sanitizing and/ or maintaining agent; determination of a concentration of a sanitizing and/ or maintaining agent; determination of an exposure time of a sanitizing and/ or maintaining agent.

9. The medical or dental sanitizing and/ or maintenance device (1) according to any one of the preceding Claims, **characterized by**
a display (9) which, based on the output data provided by the artificial intelligence module (6) and/ or appliance data provided by the database (8), is configured to visually display information about the medical or dental appliance (7) to be sanitized and/ or maintained and/ or on the sanitizing and/ or maintaining procedure to be performed by the medical or dental sanitizing and/ or maintenance device (1).

10. The medical or dental sanitizing and/ or maintenance device (1) according to any one of the preceding Claims, **characterized in that**
the medical or dental sanitizing and/ or maintenance device (1) is configured to issue an error signal if two medical or dental appliances arranged to be sanitized and/ or maintained in a shared sanitizing and/ or maintaining procedure require a different sanitizing and/ or maintaining procedure, based on the output data provided by the artificial intelligence module (6) and/ or on appliance data provided by the database (8) on these two medical or dental appliances.

11. The medical or dental sanitizing and/ or maintenance device (1) according to Claim 10, **characterized in that**
the error signal comprises the visual indication of at least one medical or dental appliance (7) which requires a different sanitizing and/ or maintaining procedure.

12. The medical or dental sanitizing and/ or maintenance device (1) according to any one of the preceding Claims, **characterized in that**
it further comprises a user interface (10) which is communicatively coupled to the artificial intelligence module (6) to train the artificial intelligence module (6).

13. A computer-implemented method (200), **characterized in that**
a trained artificial intelligence module (6)
receives (202) one or more images of a medical or dental appliance (7) to be sanitized and/ or maintained captured (201) by an imaging device (5) of a medical or dental sanitizing and/ or maintenance device (1), in particular a medical or dental sanitizing and/ or maintenance device (1) according to any one of the preceding Claims,
recognizes (203) the medical or dental appliance (7) to be sanitized and/ or maintained based on the received one or more images, and
outputs (204) data on the recognized medical or dental appliance (7) to be sanitized and/ or maintained, wherein
the control unit (4) controls (206) a sanitizing and/ or maintaining procedure of the medical or dental sanitizing and/ or maintenance device (1) based on the provided output data.

14. The computer-implemented method (200) according to Claim 13, **characterized in that** based on the output data, a database (8) provides (205) appliance data on the recognized medical or dental appliance (7) to be sanitized and/ or maintained to the control unit (4).

15. A computer program product or computer-readable storage medium comprising instructions which, when executed by a computer, cause the computer to execute the method (200) according to any of the preceding Claims 13 or 14.

16. A medical or dental sanitizing and/ or maintenance device (1) for sanitizing and/ or
maintaining a medical or dental appliance (7), comprising:
an enclosure (2) to accommodate the medical or dental appliance (7) to be sanitized and/ or maintained,
a feeding device (3) for feeding a sanitizing and/ or maintaining agent into the enclosure (2) to sanitize or maintain the medical or dental appliance,
a control unit (4) to control operation of the medical or dental sanitizing and/ or maintenance device (1), **characterized by**
an imaging device (5) which is configured and arranged to capture one or more images of the medical or dental appliance (7) to be sanitized and/ or maintained, and
an artificial intelligence module (6) which is:
communicatively coupled to the control unit (4), and to the imaging device (5) to receive the one or more images of the medical or dental appliance captured by the imaging device (5),
trained to determine the soiling of the medical or dental appliance (7) to be sanitized and/ or maintained based on the received one or more images,
and configured to output data on the soiling of medical or dental appliance (7) to be sanitized and/ or maintained, so that the control unit (4) controls a sanitizing and/ or maintaining procedure of the medical or dental sanitizing and/ or maintenance device (1) based on the provided output data on the soiling.

17. The medical or dental sanitizing and/ or maintenance device (1) according to Claim 16, **characterized in that**
it further comprises a database (8) which, based on the output data, provides sanitizing and/ or maintenance information to the control unit (4).

18. The medical or dental sanitizing and/ or maintenance device (1) according to Claim 17, **characterized in that**
the database (8) provides sanitizing and/ or maintenance information about at least on of: a sanitizing and/ or maintaining cycle to be performed; omission of a step of a predefined sanitizing and/ or maintaining cycle; determination of a duration of the sanitizing and/ or maintaining procedure; determination of an operating parameter of the sanitizing and/ or maintaining procedure; determination of a duration of a step of the sanitizing and/ or maintaining procedure; determination of a temperature in a sanitizing procedure; determination of a pressure in a sanitizing procedure; determination of a power input in a sanitizing procedure.

19. The medical or dental sanitizing and/ or maintenance device (1) according to Claim 17 or 18, **characterized in that**
the database (8) provides sanitizing and/ or maintenance information about at least on of: determination of a sanitizing and/ or maintaining agent; determination of a concentration of a sanitizing and/ or maintaining agent; determination of an exposure time of a sanitizing and/ or maintaining agent.

20. The medical or dental sanitizing and/ or maintenance device (1) according to Claim 17, 18 or 19, **characterized in that**
the database (8) provides sanitizing and/ or maintenance information about the kind of sanitizing and/ or maintenance which may be applied and/ or which must not be applied to a medical or dental appliance (7) to be sanitized and/ or maintained.

21. The medical or dental sanitizing and/ or maintenance device (1) according to any one of the preceding Claims 16 - 20, **characterized in that**
based on the received one or more images the artificial intelligence module (6) is further trained to recognize the medical or dental appliance (7) to be sanitized and/ or maintained, and
configured to output data on the recognized medical or dental appliance (7) to be sanitized and/ or maintained.

22. The medical or dental sanitizing and/ or maintenance device (1) according to any one of the preceding Claims 16 - 21, **characterized in that**
controlling the sanitizing and/ or maintaining procedure by the control unit (4) comprises at least one of: determination of a sanitizing and/ or maintaining cycle; omission of a step of a predefined sanitizing and/ or maintaining cycle; determination of a duration of the sanitizing and/ or maintaining procedure; determination of an operating parameter of the sanitizing and/ or maintaining procedure; determination of a duration of a step of the sanitizing and/ or maintaining procedure; determination of a temperature in a sanitizing procedure; determination of a pressure in a sanitizing procedure; determination of a power input in a sanitizing procedure.

23. The medical or dental sanitizing and/ or maintenance device (1) according to any one of the preceding Claims 16 - 22, **characterized in that**
controlling the sanitizing and/ or maintaining procedure by the control unit (4) comprises at least one of: determination of a sanitizing and/ or maintaining agent; determination of a concentration of a sanitizing and/ or maintaining agent; determination of an exposure time of a sanitizing and/ or maintaining agent.

24. The medical or dental sanitizing and/ or maintenance device (1) according to any one of the preceding Claims 16 - 23, **characterized by**
a display (9) which, based on the output data provided by the artificial intelligence module (6) and/ or sanitizing and/ or maintenance information provided by the database (8), is configured to visually display information about the soiling and/ or on the sanitizing and/ or maintaining procedure to be performed by the medical or dental sanitizing and/ or maintenance device (1).

25. The medical or dental sanitizing and/ or maintenance device (1) according to any one of the preceding Claims 16 - 24, **characterized in that**
the medical or dental sanitizing and/ or maintenance device (1) is configured to issue an error signal if two medical or dental appliances arranged to be sanitized and/ or maintained in a shared sanitizing and/ or maintaining procedure require a different sanitizing and/ or maintaining procedure, based on the output data provided by the artificial intelligence module (6) and/ or on sanitizing and/ or maintenance information provided by the database (8).

26. The medical or dental sanitizing and/ or maintenance device (1) according to Claim 25, **characterized in that**
the error signal comprises the visual indication of at least one medical or dental appliance which requires a different sanitizing and/ or maintaining procedure.

27. The medical or dental sanitizing and/ or maintenance device (1) according to any one of the preceding Claims 16 - 26, **characterized in that**
it further comprises a user interface (10) which is communicatively coupled to the artificial intelligence module (6) to train the artificial intelligence module (6).

28. A computer-implemented method (200), **characterized in that**
a trained artificial intelligence module (6)
receives (202) one or more images of a medical or dental appliance (7) to be sanitized and/ or maintained captured (201) by an imaging device (5) of a medical or dental sanitizing and/ or maintenance device (1), in particular a medical or dental sanitizing and/ or maintenance device (1) according to any one of the preceding Claims 16 - 27,
determines (203) the soiling of the medical or dental appliance (7) to be sanitized and/ or maintained based on the received one or more images, and
outputs (204) data on the soiling, wherein
the control unit (4) controls (206) a sanitizing and/ or maintaining procedure of the medical or dental sanitizing and/ or maintenance device (1) based on the provided output data on the soiling.

29. The computer-implemented method (200) according to Claim 28, **characterized in that** based on the output data, a database (8) provides (205) sanitizing and/ or maintenance information to the control unit (4).

30. A computer program product or computer-readable storage medium comprising instructions which, when executed by a computer, cause the computer to execute the method (200) according to any of the preceding Claims 28 or 29.
